# EUROPEAN PATENT SPECIFICATION

(11) **EP 3 328 451 B1**
(45) Date of publication and mention of the grant of the patent: **11.09.2019**
(21) Application number: 16744759.8
(22) Date of filing: 28.07.2016
(51) Int. Cl.: A61L 27/10, A61L 27/30, A61L 31/02, A61L 31/08

(54) **IMPLANTS WITH AT LEAST ONE OSSEOINTEGRATIVE AND ANTI-MICROBIAL SURFACE (MULTI)LAYER STRUCTURE**
IMPLANTATE MIT MINDESTENS EINER OSSEOINTEGRATIVEN UND ANTIMIKROBIELLEN OBERFLÄCHEN-(MEHRFACH)SCHICHTSTRUKTUR
IMPLANTS COMPRENANT AU MOINS UNE STRUCTURE MONO OU MULTICOUCHE ANTIMICROBIENNE À INTÉGRATION OSSEUSE

(30) Priority: 28.07.2015 EP 15178659
(43) Date of publication of application: 06.06.2018
(73) Proprietor: CeramTec GmbH, 73207 Plochingen (DE)
(72) Inventor: PORPORATI, Alessandro Alan, 73207 Plochingen (DE); RASCHKE, Marita, 71229 Leonberg (DE); STREICHER, Professor Robert, 8835 Feusisberg (CH); KUNTZ, Meinhard, 73733 Esslingen (DE); PREUSS, Roman, 73230 Kirchheim unter Teck (DE); GOTTWIK, Lukas, 73087 Bad Boll (DE)
(74) Representative: Fehrenbacher, Eckhard Anton
(86) International application number: PCT/EP2016/067972
(87) International publication number: WO 2017/017166

(56) References cited:
- EP-A1- 2 810 665
- JP-A- 2013 132 612

## Description

The present invention relates implants comprising metallic and / or ceramic components, as defined by claims 1-7, having improved osseointegrative and anti-microbial properties, the metallic and / or ceramic components are suitable for a cementless fixation in the human body, for example the acetabular region of the human pelvis. The present invention also relates to a method for producing the implants, as defined by claims 8 and 9.

Nowadays metal shells with osseointegrative surface structures are necessary for a cementless fixation of ceramic inserts in the acetabular region of the human pelvis. Possibilities to fix ceramic implants directly into bone are described for example in EP 1268364 A1 and EP2810665 A1, wherein a ceramic body is layered with a surface structure having open pores.

One of the major causes of a total hip or knee replacement revision is the septic loosening due to formation of a biofilm on the implant surface. Unfortunately, a rough surface, which is necessary for promoting bone ingrowth of an implant, may increase the probability of the formation of a bacteria biofilm.

Therefore, it is an object of the present invention to provide metallic and / or ceramic components, in particular in the field of medical technology, being less susceptible for the formation of (bacteria) biofilms.

This object is solved by the implant of the present invention which comprises an osseointegrative and anti-microbial surface (multi)layer structure, wherein a surface layer of the surface (multi)layer structure comprises lanthanum aluminate according to claim 1. It also enables the direct implantation of the metallic and / or ceramic component due to the addition of the porous osseointegrative surface (multi)layer structure comprising lanthanum aluminate to the backside of a ceramic substrate.

The implant comprising the osseointegrative and anti-microbial surface (multi)layer structure of the present invention is able to decrease the bacteria biofilm growth rate due to the presence of lanthanum aluminate in the osseointegrative and anti-microbial surface (multi)layer structure on the metallic or ceramic surface of the ceramic component. The surface (multi)layer structure may have also osteoinductive properties and therefore may promote the growth, maturation and activity of osteoblasts. The lanthanum cation may be particularly efficient because of its high charge density, which may reduce the net negative charge on the bacteria, resulting in an anti-microbial activity.

The term metallic and / or ceramic component as used in the present invention comprises components composed of metals only or ceramic materials only, but also components being mainly composed of ceramics and having a metallic surface for example. Preferred metallic and / or ceramic components of the present invention are ceramic components used in the field of medical technology.

The metallic and / or ceramic component of the present invention is to be used in the field of medical technology and is comprised in an implant, for example a cup in hip arthroplasty or a cage for the spine.

The implant of the present invention comprises an osseointegrative and anti-microbial surface (multi)layer structure on its surface comprising at least one osseointegrative and anti-microbial layer. The osseointegrative and anti-microbial surface (multi)layer structure preferably comprises several osseointegrative and anti-microbial layers, for example up to 30 layers. Preferably, the osseointegrative anti-microbial surface (multi)layer structure comprises from 1 to 5 osseointegrative layers.

The osseointegrative and anti-microbial surface (multi)layer structure of the present invention comprises a ceramic base material and lanthanum aluminate. The term lanthanum aluminate comprises any known lanthanum aluminate compound. The preferred compound is lanthanum hexaaluminate. The lanthanum aluminate is preferably present in platelet-form within the osseointegrative and anti-microbial surface (multi)layer structure. The content of the lanthanum aluminate may be in the range of from 1 wt% to 50 wt%, preferably 1 wt% to 20 wt% based on the total weight of the surface (multi)layer structure.

The ceramic base material is selected from the group consisting of alumina based ceramics, zirconia based ceramics, preferably alumina based ceramics and zirconia based ceramics, more preferably a zirconia-platelet toughened alumina ceramic. The zirconia content in the zirconia-platelet toughened alumina ceramic is up to 25 wt% based on the total weight of the surface (multi)layer structure.

The osseointegrative and anti-microbial surface (multi)layer structure may exhibit a lanthanum gradient by means of different lanthanum contents in different layers of the osseointegrative and anti-microbial surface (multi)layer structure with the aim to gradually release the residual stresses in the surface (multi)layer structure. In a preferred embodiment, the lanthanum content increases with the layer number, wherein the first / innermost layer has the lowest lanthanum content and the last / outermost layer has the largest lanthanum content. The outermost layer has to be seen as the layer coming in direct contact with the surrounding tissue or bone of the human body.

The amount of the lanthanum aluminate in the innermost layer of the respective lanthanum gradient structure is less than 5 wt%, preferably equal to or less than 1 wt% based on the total weight of the surface (multi)layer structure, with the proviso that the lanthanum content is not zero. The outermost layer comprises lanthanum aluminate in an amount of less than 25 wt%, preferably between 15 and 20 wt% based on the total weight of the surface (multi)layer structure.

The porosity of the osseointegrative and anti-microbial surface (multi)layer structure, that is the number of pores per unit volume, may be between 25% and 90%, preferably between 25% and 70%. The diameter of the pores of between is between 1 micron and 1000 microns, preferably between 20 microns and 600 microns. The number of pores per unit volume, their size, that is, its diameter, and its shape can advantageously be determined by the selection of a suitable pore-forming substance.

The thickness of the whole layer is between about 0.02 mm and 10 mm, preferably between 0.1 mm and 2 mm.

For the production of a ceramic component with an osseointegrative and anti-microbial surface (multi)layer structure, such as a an anti-microbial osseointegrative implant, high content lanthanum alumina/zirconia-based ceramic slurry can be deposited on a green substrate by using the method as described for example in EP 1268364 A1, where the substrate is formed as a green body of an inorganic material and to the substrate in the state of the green body, a suspension the same inorganic material constituting the substrate, or other material is applied. This suspension contains the layer material and additionally may contain a pore-forming substance or the pore-forming substance can also be applied separately. Only after applying the layer is a common heat treatment of substrate and layer by drying and sintering to produce a monolithic molding. The method for producing the substrate does not generally differ from those known from the prior art.

During the sintering, the green substrate achieves the full density; on the other hand the surface (multi)layer structure remains micro-porous, because of lower sintering activity caused by the high volume platelets content; and macro porous, because of pore forming or foaming agents. The diameter of the micro-pores is of between 0.05 micron to 5 micron, but preferably at submicron-level, from 0,1 micron to 1 micron, whilst the macro-pores have a diameter between 1 and 100 micron, preferably between 10 and 500 micron, with the proviso that the macro-pores are larger in diameter than the micro-pores. Moreover, it has to be understood that the micropores should look like "caves" into the material and the macropores like "valleys".

The alumina-based ceramic slurry comprises alumina, zirconia, lanthanum oxide and yttrium oxide. During the sintering stage, the solid reaction between alumina and lanthanum oxide undergoes forming the lanthanum aluminate, in particular the lanthanum hexaaluminate.

In order to achieve a macro-porous osseointegrative and osteoinductive surface (multi)layer structure the respective ceramic slurry may also comprise (organic) additives such as pore forming agents or foaming agents based on the respective method for forming a porous layer. Such additives may be present in amounts of from 0.05 to 10 wt% based on the total weight of the surface (multi)layer structure composition. While the pore forming agent is pyrolized during the sintering process, the foaming agent is used in foaming process both leading to a porous osseointegrative and osteoinductive layer(s) having a porosity as disclosed above.

Thus, the porosity may be enhanced with organic pore-forming agents in the sintering process or with pore-forming agents or foaming agents and foaming procedures, respectively.

As pore-forming substances, in particular organic compounds, for example, starches, cellulose or waxes, and natural and synthetic polymers, which evaporate during the thermal treatment of the substrate and the deposited thereon layer gasify to consume or burn, thereby forming the pores are used.

The ceramic slurry may comprise further additives commonly used in the field of ceramic technology, such as binder.

Alternatively, it is also possible to apply the osseointegrative and anti-microbial layer(s) on a substrate in sintered state. Thereby, the surface (multi)layer structure may be deposited on the sintered ceramic substrate or *ad-hoc* prepared metallic substrate by vapour- or plasma-deposition processes.

The osseointegrative and anti-microbial surface (multi)layer structure may be also grown on pre-existing macro-porous surface which might be ceramic foam or metallic osseointegrative surface. Micro- and macro- porosities are produced for an improved osseointegration and osseoinduction.

The osseointegrative and anti-microbial surface (multi)layer structure may also be deposited on structured surfaces of the ceramic component. A structured surface in the sense of the present invention is for example a porous surface. The structuring of the ceramic component surface may be produced by milling processes, turning processes or a combination of both. Another method for producing a structured surface is the method of ceramic injection molding. The structuring of the metallic or ceramic component surface may be produced by for example by additive manufacturing.

Alternatively, pore forming agents which are pyrolized during the sintering or foaming agents for foaming processes can be added to the substrate material in order to produce a structured surface.

Other methods for producing structured surfaces are grinding or electro discharge machining processes.

## Claims

1. An implant comprising a metallic and/or ceramic component and an osseointegrative and anti-microbial surface (multi)layer structure on its surface comprising at least one osseointegrative and anti-microbial layer wherein the osseointegrative and anti-microbial layer comprises lanthanum aluminate.

2. The implant of claim 1, wherein the osseointegrative and anti-microbial surface (multi)layer structure comprises several osseointegrative and anti-microbial layers.

3. The implant of claims 1 or 2, wherein the lanthanum aluminate is present in platelet-form.

4. The implant of claim 2, wherein the osseointegrative and anti-microbial layers of the surface (multi)layer structure have different lanthanum contents.

5. The implant of claim 4, wherein the lanthanum content increases with the layer number so that the outermost layer has the highest lanthanum content.

6. The implant of claims 1 to 5, wherein the surface of the ceramic component is structured.

7. The implant of claim 6, wherein the structuring of the surface is carried out by a process selected from the group consisting of a milling process, a turning process, a combination of a milling and a turning process, ceramic injection molding, a foaming process due to the addition of a foaming agent, a pore forming process during sintering due to the addition of a pore forming agent, a grinding process electro discharge machining, and additive manufacturing.

8. A method for producing an implant in accordance with claims 1 to 7, wherein a ceramic slurry comprising lanthanum aluminate is applied to a substrate in green state

9. A method for producing the implant in accordance with claims 1 to 7, wherein the osseointegrative and anti-microbial surface (multi)layer structure is applied to a substrate in sintered state.

10. The method of claim 9, wherein the osseointegrative surface (multi)layer structure is applied by vapour- or plasma deposition.

## Patentansprüche

1. Implantat, umfassend eine metallische und/oder keramische Komponente und einer osseointegrativen und antimikrobiellen Oberflächen(mehr)schichtstruktur auf ihrer Oberfläche, die wenigstens eine osseointegrative und antimikrobielle Schicht umfasst, wobei die osseointegrative und antimikrobielle Schicht Lanthanaluminat umfasst.

2. Implantat nach Anspruch 1, wobei die osseointegrative und antimikrobielle Oberflächen(mehr)schichtstruktur einige osseointegrative und antimikrobielle Schichten umfasst.

3. Implantat nach einem der Ansprüche 1 oder 2, wobei das Lanthanaluminat in einer Plättchenform vorliegt.

4. Implantat nach Anspruch 2, wobei die osseointegrativen und antimikrobiellen Schichten der Oberflächen(mehr)schichtstruktur verschiedene Lanthangehälter aufweisen.

5. Implantat nach Anspruch 4, wobei der Lanthangehalt mit der Schichtanzahl derart zunimmt, dass die äußerste Schicht den höchsten Lanthangehalt aufweist.

6. Implantat nach einem der Ansprüche 1 bis 5, wobei die Oberfläche der keramischen Komponente strukturiert ist.

7. Implantat nach Anspruch 6, wobei die Strukturierung der Oberfläche durch einen Vorgang durchgeführt wird, der aus der Gruppe bestehend aus einem Fräsvorgang, einem Drehvorgang, einer Kombination aus einem Fräs- und Drehvorgang, Keramikspritzgießen, einem Schäumungsvorgang aufgrund der Zugabe eines Schäumungsmittels, einem Porenausbildungsvorgang während eines Sinterns aufgrund der Zugabe eines Porenausbildungsmittels, einem Schleifvorgang durch funkenerosives Abtragen und additivem Fertigen ausgewählt ist.

8. Verfahren zum Herstellen eines Implantats nach den Ansprüchen 1 bis 7, wobei eine Keramikaufschlämmung, Lanthanaluminat umfassend, auf ein Substrat in rohem Zustand aufgetragen wird.

9. Verfahren zum Herstellen des Implantats nach den Ansprüchen 1 bis 7, wobei die osseointegrative und antimikrobielle Oberflächen(mehr)schichtstruktur auf ein Substrat in einem gesinterten Zustand aufgetragen wird.

10. Verfahren nach Anspruch 9, wobei die osseointegrative Oberflächen(mehr)schichtstruktur durch Aufdampfen oder plasmaunterstütztes CVD aufgetragen wird.

## Revendications

1. Implant comprenant un composant métallique et/ou céramique et une structure (multi)couche de surface ostéo-intégrative et antimicrobienne comprenant au moins une couche ostéo-intégrative et antimicrobienne, dans lequel la couche ostéo-intégrative et antimicrobienne comprend de l'aluminate de lanthane.

2. Implant selon la revendication 1, dans lequel la structure (multi)couche de surface ostéo-intégrative et antimicrobienne comprend plusieurs couches ostéo-intégratives et antimicrobiennes.

3. Implant selon la revendication 1 ou 2, dans lequel l'aluminate de lanthane est présent sous forme de plaquettes.

4. Implant selon la revendication 2, dans lequel les couches ostéo-intégratives et antimicrobiennes de la structure (multi)couche de surface ont différentes teneurs en lanthane.

5. Implant selon la revendication 4, dans lequel la teneur en lanthane augmente avec le nombre de couches de sorte que la couche la plus externe a la teneur en lanthane la plus élevée.

6. Implant selon les revendications 1 à 5, dans lequel la surface du composant céramique est structurée.

7. Implant selon la revendication 6, dans lequel la structuration de la surface est réalisée par un procédé choisi dans le groupe constitué par un procédé de broyage, un procédé de rotation, une combinaison d'un procédé de broyage et de rotation, un moulage par injection de céramique, un procédé de moussage dû à l'ajout d'un agent moussant, un procédé de formation de pores lors du frittage dû à l'ajout d'un agent de formation de pores, un procédé de meulage, d'usinage par électro-décharge et de fabrication d'additifs.

8. Procédé de production d'un implant selon les revendications 1 à 7, dans lequel une suspension de céramique comprenant de l'aluminate de lanthane est appliquée sur un substrat à l'état vert.

9. Procédé de production de l'implant selon les revendications 1 à 7, dans lequel la structure (multi)couche de surface ostéo-intégrative et antimicrobienne est appliquée sur un substrat à l'état fritté.

10. Procédé selon la revendication 9, dans lequel la structure (multi)couche de surface ostéo-intégrative est appliquée par dépôt de vapeur ou de plasma.
